# EUROPEAN PATENT SPECIFICATION

(11) **EP 1 552 852 B1**
(45) Date of publication and mention of the grant of the patent: **29.04.2009**
(21) Application number: 03784615.1
(22) Date of filing: 08.08.2003
(51) Int. Cl.: A61K 48/00, C12N 15/861, A61P 35/00

(54) **COMPOSITIONS FOR INHIBITION OF PROLIFERATION AND INFILTRATION OF BRAIN TUMOR CELLS BY EXPRESSION OF AMPA-TYPE GLUTAMATE RECEPTOR SUBUNIT**
ZUSAMMENSETZUNGEN ZUR HEMMUNG DER PROLIFERATION UND INFILTRATION VON GEHIRNTUMORZELLEN DURCH EXPRESSION DER AMPA-TYP GLUTAMAT-REZEPTOR-UNTEREINHEIT
COMPOSITIONS POUR L'INHIBITION DE LA PROLIFERATION ET DE L'INFILTRATION DE CELLULES TUMORALES CEREBRALES PAR L'EXPRESSION DE LA SOUS-UNITE DE TYPE AMPA DU RECEPTEUR DU GLUTAMATE

(30) Priority: 08.08.2002 JP 2002232086
(43) Date of publication of application: 13.07.2005
(73) Proprietor: Japan Science and Technology Agency, Kawaguchi-shi, Saitama 332-0012 (JP)
(72) Inventor: OZAWA, Seiji, Maebashi-shi, Gunma 371-0033 (JP); ISHIUCHI, Shogo, Maebashi-shi, Gunma 371-0844 (JP)
(74) Representative: Harding, Charles Thomas
(86) International application number: PCT/JP2003/010143
(87) International publication number: WO 2004/014433

(56) References cited:
- ISHIUCHI S ET AL: "Extension of glial processes by activation of Ca2+-permeable AMPA receptor channels" NEUROREPORT, RAPID COMMUNICATIONS OF OXFORD, OXFORD, GB, vol. 12, no. 4, 26 March 2001 (2001-03-26), pages 745-748, XP002974830 ISSN: 0959-4965
- RZESKI W ET AL: "Glutamate antagonists limit tumor growth" PROCEEDINGS OF THE NATIONAL ACADEMY OF SCIENCES OF USA, NATIONAL ACADEMY OF SCIENCE, WASHINGTON, DC, US, vol. 98, no. 11, 22 May 2001 (2001-05-22), pages 6372-6377, XP002968567 ISSN: 0027-8424
- MAAS S ET AL: "Underediting of glutamate receptor GluR-B mRNA in malignant gliomas" PROCEEDINGS OF THE NATIONAL ACADEMY OF SCIENCES OF USA, NATIONAL ACADEMY OF SCIENCE, WASHINGTON, DC, US, vol. 98, no. 25, 4 December 2001 (2001-12-04), pages 14687-14692, XP002974829 ISSN: 0027-8424
- ISHIUCHI, SHOGO: "A crucial role of Ca2+-permeable AMPA receptors in human glioblastoma cells" 6JIKKEN IGAKU , 20(18), 2651-2653 CODEN: JIIGEF; ISSN: 0288-5514, 2002, XP008070096
- ISHIUCHI S ET AL: "Blockage of Ca2+-permeable AMPA receptors suppresses migration and induces apoptosis in human glioblastoma cells" NATURE MEDICINE, NATURE AMERICA, NEW YORK, US, vol. 8, no. 9, September 2002 (2002-09), pages 971-978, XP002970007 ISSN: 1078-8956
- RZESKI W. ET AL.: 'Glutamate antagonists limit tumor growth' PROC. NATL. ACAD. SCI. USA vol. 98, no. 11, 22 May 2001, pages 6372 - 6377, XP002968567
- MASS S. ET AL.: 'Underediting of glutamate receptor GluR-B mRNA in malignant gliomas' PROC. NATL. ACAD. SCI. USA vol. 98, no. 25, 04 December 2001, pages 14687 - 14692, XP002974829
- ISHIUCHI S. ET AL.: 'Extension of glial processes by activation of Ca2+-permeable AMPA receptor channels' NEUROREPORT vol. 12, no. 4, 26 March 2001, pages 745 - 748, XP002974830

## Description

### Technical Field

The present invention relates to compositions for inhibiting proliferation and invasion of brain tumor cells by regulating Ca²⁺ permeability in developing animal brain tumor cells, in particular, a composition for inhibiting proliferation and invasion of brain tumor cells by regulating Ca²⁺ permeability by an AMPA-type glutamate receptor subunit, and a method for measuring proliferation/invasion activity of brain tumor cells by detecting/measuring the expression of an AMPA-type glutamate receptor subunit in developing animal brain tumor cells.

### Background Art

Glutamic acid is a major excitatory neurotransmitter in the central nervous system of higher animals, and glutamate receptors, which are receptors of glutamic acid, play a central role in excitatory synaptic transmission in the central nervous system. Further, it is known that glutamate receptors are involved in synaptic plasticity thought to be a cellular-level basis of memory and learning, and synaptic plasticity during the developmental period, in other words, the experience-dependent formation of neural network. In addition, it is suggested that glutamate receptors are involved in neuronal cell death under pathological conditions such as ischemia.

Glutamate receptors are roughly classified, by their structure and signaling mechanism, into ionotropic glutamate receptors, which have an ion channel for fast synaptic transmission, and metabotropic glutamate receptors, which transmit signals indirectly by conjugating G protein. Ionotropic glutamate receptors are receptor-ion channel complexes which open their cation channels in response to the binding of glutamic acid, and responsible for fast excitatory synaptic transmission in the central nervous system (CNS) of higher animals.

According to their reactivity to specific agonists or antagonists and electrophysiologic properties of ion channels they contain, ionotropic receptors are roughly classified into NMDA (N-methyl-D-aspartic acid) receptors being sensitive to NMDA, and non-NMDA receptors being insensitive to NMDA. When agonists bind to them, NMDA receptors permit Na⁺, K⁺ and Ca²⁺ to pass through them. According to the difference between their reactivities to kainate receptors and AMPA (α-amino-3-hydroxy-5-methyl-4-isoxazole propionic acid), non-NMDA receptors are classified into subtypes of kainate receptors and AMPA receptors, and when agonists bind to them, they permit Na⁺ and K⁺ to pass through them.

AMPA-type glutamate receptors (AMPARs) mediate fast neurotransmission in almost all excitatory synapses in the central nervous system (CNS) (Trends Neurosci., 16, 359-365, 1993; Annu. Rev. Neurosci., 17, 31-108, 1994; Prog. Neurobiol., 54, 581-618, 1998). Conventionally, the receptors have been thought to be exist only in neurons, but recently, it is revealed that they are also expressed in the majority of glial cells (Trends Pharmacol. Sci., 21, 252-258, 2000).

It is known that AMPA receptors (AMPARs) are composed of subunits selected from a set of four proteins GluR1 to GluR4. The Ca²⁺ permeability of AMPARs depends on their subunit composition.

Receptors possessing GluR2 subunits exhibit little permeability to Ca²⁺, whereas those lacking GluR2 exhibit high Ca²⁺ permeability. Abundance of the GluR2 subunit has been shown to decrease Ca²⁺ permeability (Trends Neurosci., 16, 359-365, 1993; Annu. Rev. Neurosci., 17, 31-108, 1994; Prog. Neurobiol. , 54, 581-618, 1998). The unique properties of GluR2 can be traced to a single amino acid residue in the second hydrophobic segment (M2). This residue is arginine (R) in GluR2, whereas the corresponding site is occupied by glutamine (Q) in the other subunits. When the arginine in this critical site (Q/R site) is replaced with glutamine, the homomeric receptors assembled from the mutant GluR2 (Q) show high Ca²⁺ permeability.

A mechanism wherein one amino acid residue in a hydrophobic segment (M2) of GluR2, among the subunits of AMPAR, is replaced with arginine (R) in living organisms, is known as a phenomenon which arises after gene transcription called "RNA editing". In other words, one base is substituted after a gene (DNA) is transcribed to mRNA, resulting that one amino acid residue is substituted at protein level as well. It is a peculiar phenomenon wherein glutamic acid (Q) is encoded in a gene, but actually, it is substituted with arginine (R). However, this conversion decreases Ca²⁺ permeability.

As mentioned above, because glutamate receptors act as a receptor for neurotransmitters, studies of glutamate receptors have been keenly conducted mainly in regard to psychological disorder, amyotrophic lateral sclerosis (ALS), and in the field of memory/learning, and various findings have been obtained and therapeutic drugs, etc., have been developed. As a result of such research and development, methods for identifying genes involved in neurodegenerative diseases such as Parkinson's disease, Huntington's chorea, amyotrophic lateral sclerosis, multiple sclerosis, Alzheimer's disease, stroke or epilepsy, and therapeutic drugs for treating animals suffering from neurodegenerative diseases, etc., have been developed.

On the other hand, glioblastoma, oligodendroglioma, meningioma, etc., are well known as brain tumors. In particular, glioblastoma is the most popular and most malignant tumor in the central nervous system (CNS). It is known that this kind of tumor is highly migratory and invasive (Pathology and Genetics of Tumours of the Nervous Systems, 29-39, 2000 (IARC press, Lyon, France)). As migratory cells show preferential movement along a dense and myelinated pathway, and spread widely in the CNS (Neurol. Med. Chir., 34, 91-94, 1994; Neurol. Med. Chir., 33, 425-428, 1993; Neuropathology, 17, 186-188, 1997), surgical procedures have not led to good results.

In addition, cellular proliferation and migration, which are two major characteristics of malignant glioma, are also found in neural progenitor cells from which glioma originates (Glial Cell Development, 209-220, 1996 (BIOS Scientific Publishers, Oxford, UK.); Glia, 15, 222-230, 1995). As glioma cells are responsive to various external stimuli such as neurotransmitters, hormones, and growth factors (Trends Pharmacol. Sci., 21, 252-258, 2000), malignant characteristics of glioblastoma can be controlled at least partially through the activation of surface receptors.

Recently, it is reported that glutamate receptors are expressed not only in neural progenitor cells but also in glioma (J. Neurosci., 17, 227-240, 1997; Glia, 10, 149-153, 1994; J. Neurosci., 16, 519-530, 1996; Eur. J. Neurosci., 10, 2153-2162, 1998; J. Neurosci. Res., 46, 164-178, 1996). However, most of their pathophysiological significance remains obscure.

Relating to the possible treatment of tumours, for example a neuroblastoma, Proc.Natl.Acad.Sci.,USA,2001, vol.98,6372-6377, discloses the use of glutamate antagonists, for example GYKI52466, an AMPA antagonist.

The object of the present invention is to provide a composition for inhibiting proliferation and invasion of brain tumor cells by regulating Ca²⁺ permeability in developing animal brain tumor cells, in particular, a composition for inhibiting proliferation and invasion of brain tumor cells by regulating Ca²⁺ permeability by an AMPA-type glutamate receptor subunit, and further, a method for measuring proliferation/invasion activity of brain tumor cells by detecting/measuring the expression of an AMPA-type glutamate receptor subunit in developing animal brain tumor cells.

In the process of analyzing the role of calcium-permeable AMPA receptors in glial cells by using an established culture system of brain cells, the present inventors have found and reported that formation of cellular processes is promoted when GluR2 (Q) genes having strong calcium permeability are forced to express, whereas processes are retracted and cells are flattened when GluR2 (GluR2 (R)) genes having calcium impermeability are introduced (NeuroReport 12, (2001), 745-748).

Further, the present inventors have suspected that calcium-permeable AMPARs (AMPA receptors) are involved in the invasion property of tumor cells, based on the fact that AMPARs are expressed in human glioma cells and further, fusiform cells wherein GluR2 (Q) genes are forced to express are morphologically similar to glioma cells which migrate while becoming stratified in a culture flask, and to tumor cells which run through myelin fibers in white matter, which are frequently found in extirpated samples. In addition, as a result of further analysis, the present inventors have found that (1) GluR1 and/or GluR4 subunits are ubiquitously expressed in human glioblastoma cells, and act as Ca²⁺-permeable AMPAR, (2) when GluR2 (GluR2 (R)) genes are introduced into the glioblastoma cells and such endogenous Ca²⁺-permeable AMPARs are converted into Ca²⁺-impermeable AMPARs, migration is inhibited in tumor cells and apoptosis is induced, and (3) on the contrary, when Ca²⁺-permeable AMPARs are overexpressed, not only the proliferation of tumor cells but also migration behavior is increased, and the present invention has been completed.

In other words, the present invention pertains to the inhibition of proliferation and invasion of brain tumor cells by regulating Ca²⁺ permeability in developing animal brain tumor cells by glutamate receptor subunits. The regulation of Ca²⁺ permeability by glutamate receptor subunits can be conducted by introducing a gene of an AMPA-type glutamate receptor subunit GluR2 into a brain tumor cell. Further, the present invention provides a method for measuring proliferation/invasion activity of brain tumor cells by detecting/measuring the expression of glutamate receptor subunits in developing animal brain tumor cells.

### Disclosure of the Invention

The present invention relates to a composition for inhibiting proliferation and invasion of brain tumor cells by regulating Ca²⁺ permeability by an AMPA-type glutamate receptor subunit being expressed in a developing animal brain tumor cell ("1"), the composition for inhibiting proliferation and invasion of brain tumor cells according to "1", wherein the regulation of Ca²⁺ permeability by a glutamate receptor subunit is conducted by introducing a gene of an AMPA-type glutamate receptor subunit GluR2 (R) into a developing animal brain tumor cell and expressing the gene ("2"), the composition for inhibiting proliferation and invasion of brain tumor cells according to "2" , wherein the gene of an AMPA-type glutamate receptor subunit GluR2 (R) is a cDNA of an AMPA-type glutamate receptor subunit GluR2 (R) ("3"), the composition for inhibiting proliferation and invasion of brain tumor cells according to "2" or "3", wherein the gene of an AMPA-type glutamate receptor subunit GluR2 (R) is introduced into a developing animal brain tumor cell by an expression vector, and is expressed ("4"), the composition for inhibiting proliferation and invasion of brain tumor cells according to "4", wherein the expression vector is a vector using an adenovirus ("5"), and the composition for inhibiting proliferation and invasion of brain tumor cells according to any one of "1" to "5", wherein the brain tumor cell is a glioblastoma ("6").

Also described herein are a gene expression vector for treating a brain tumor wherein a gene of an AMPA-type glutamate receptor subunit GluR2 (R) is incorporated into a gene introduction/expression vector for a brain tumor cell ("7"), the gene expression vector for treating a brain tumor according to "7", wherein the expression vector is an adenoviral vector ("8"), and a gene introduction kit for treating a brain tumor containing the gene expression vector for treating a brain tumor according to "7" or "8" ("9").

The present invention further relates to a method for measuring proliferation/invasion activity of brain tumor cells wherein the expression of an AMPA-type glutamate receptor subunit (GluR2 (R)) in a developing animal brain tumor cell is detected/measured ("10"), and the method for measuring proliferation/invasion activity of brain tumor cells according to "10", wherein the detection/measurement of the glutamate receptor subunit is detection/measurement of an AMPA-type glutamate receptor subunit GluR2 (R) ("11").

### Brief Description of Drawings

Fig. 1 is a set of photographs showing the expression of AMPA receptors in human glioblastoma cells in the present invention.
Fig. 2 is a set of photographs showing the change of [Ca²⁺]i induced by AMPAs in cultured tumor cells in the present invention.
Fig. 3 is a set of photographs showing the effect of the expression of AMPA receptor subunits mediated by adenovirus in the present invention.
Fig. 4 is a set of views showing the effect of the expression of GluR2 and GluR2 (Q) on the migration of cells in the present invention.
Fig. 5 is a set of photographs showing the effect of the expression of GluR2 on the tumor transplant in the present invention.
Fig. 6 is a set of views showing the effect of the manipulation of AMPA receptors on tumor growth in the present invention.

### Best Mode of Carrying Out the Invention

The present invention comprises compositions for inhibition of proliferation and invasion of brain tumor cells by regulating Ca²⁺ permeability in developing animal brain tumor cells by expressing glutamate receptor subunits. Examples of the developing animal brain tumor cell include glioblastoma, oligodendroglioma and meningioma. The regulation of Ca²⁺ permeability by glutamate receptor subunits can be conducted by introducing a gene of an AMPA-type glutamate receptor subunit GluR2 (R) into a developing animal brain tumor cell, and expressing the gene. As the gene of an AMPA-type glutamate receptor subunit GluR2 (R), a cDNA of an AMPA-type glutamate receptor subunit GluR2 (R) can be used, but the gene is already known (Science 249, 1580-1585, 1990), and the base sequence of the gene can be retrieved from GenBank data base as Accession No. M38061.

For the introduction of a gene of an AMPA-type glutamate receptor subunit GluR2 (R) into developing animal brain tumor cells in the present invention, appropriate and known gene introduction methods can be used. An adenovirus expression vector can be used for preferable introduction methods. As such adenovirus expression vector, an adenoviral vector used for transient expression (Science, 259, 988-990, 1993) is exemplified. Adenoviral vectors have extremely high affinity to glial cells in the central nervous system (CNS), and when they are injected into cerebellar cortex, they can express a GluR2 gene in Bergmann glia selectively. In addition, as for adenoviral vectors, gene introduction vectors employing Cre/loxP expression control system (Nucl. Acids Res., 23, 3816-3821, 1995) can be used. One of the reasons for using Cre/loxP system is the following merit: as Cre does not exist in an infection only with AxCALNLGluR2, a condition wherein no GluR2 (R) is expressed can be created, and such condition can be used as control for excluding the influence of viral infection.

In the present invention, a gene of an AMPA-type glutamate receptor subunit GluR2 (R) can be incorporated into a gene expression vector such as adenoviral vectors to prepare a gene introduction kit for treating brain tumors, and such kit can be used for the treatment of brain tumors.

As another embodiment of the present invention, the method of the present invention makes it possible to measure proliferation/invasion activity of brain tumor cells by detecting/measuring the expression of glutamate receptor subunits in developing animal brain tumor cells. In brain tumors such as glioblastoma in the central nervous system (CNS), subunits composed of a set of four proteins GluR1 ∼ 4 are expressed, and the Ca²⁺ permeability or impermeability of cells depends on the composition of the subunits being expressed. This Ca²⁺ permeability or impermeability of cells results in the difference in proliferation/invasion activity of brain tumor cells. The expression of subunit GluR1 or GluR4 makes cells Ca²⁺-permeable, and promotes proliferation/invasion of brain tumor cells, whereas the expression of subunit GluR2 (R) makes cells Ca²⁺-impermeable, and inhibits proliferation/invasion of brain tumor cells.

Therefore, proliferation/invasion activity of brain tumor cells can be measured by detecting/measuring glutamate receptor subunits expressed in brain tumor cells. For the detection/measurement of glutamate receptor subunits, known means of detection/measurement can be appropriately used. In order to measure genes of subunits, a probe for detecting a gene of each subunit can be used. In the detection/measurement of the expression of subunits, it is preferable to construct and use an antibody which specifically binds to each subunit. As an antibody which specifically binds to a glutamate receptor subunit, either of polyclonal or monoclonal antibody can be used, and such antibodies can be prepared by ordinary methods.

The present invention will be described more specifically with examples, but the technical scope of the present invention is not limited to these examples.

### [Expression of glutamate receptor subunits, introduction of subunit GluR2 (R), and proliferation and invasion of tumor cells] (Expression of GluRs in glioblastoma surgical samples and cell cultures)

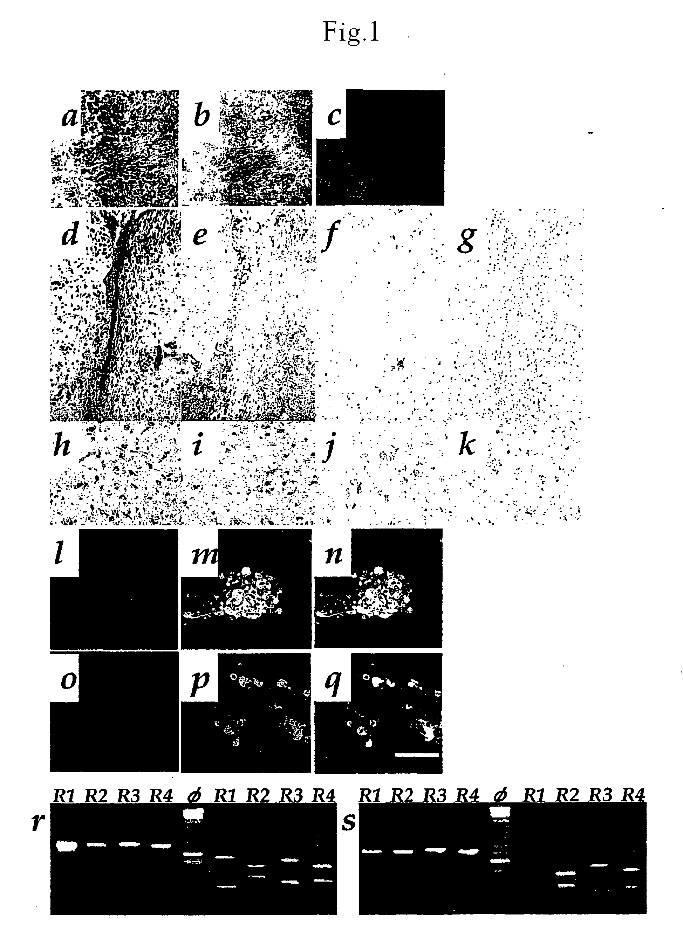

The present inventors examined the expression of AMPAR subunit GluR1 in in situ hybridization study of surgical samples (see Fig. 1). Fig. 1a shows representative histology of a glioblastoma surgical sample, demonstrating invasive tumor cells. The hybridized signals with the antisense riboprobe of GluR1 were detected in migrating and proliferating undifferentiated cells (Fig. 1b). In the serial section, the expression of GluR1 protein immunostained by the affinity purified GluRl antibody was detected in tumor cells (Fig. 1c).

The present inventors further examined the expression of AMPAR subunits in paraffin-embedded glioblastoma surgical samples (n = 16) by immunohistochemical staining with selected antibodies against GluR1, GluR2 (R), GluR2/3 and GluR4 (Table 1). Expression of GluR1 protein was found in all surgical samples examined (n = 16) . In these samples, GluR1 protein was expressed in the majority of tumor cells (52 ± 21%, n = 16). In 14 of 16 glioblastoma surgical samples, GluR2 (R) expression was observed in the minority of cells (21 ± 7%, n = 14). The staining with anti-GluR2/3 antibody was observed in 14 of 16 surgical samples (31 ± 5%, n = 14). The tumor cells were also positive for GluR4 in all surgical samples (55 ± 7%, n = 16).

Fig. 1d to g show an example of serial sections from an original surgical sample treated with antibodies against GluR1, GluR2 (R) and GluR4. In these sections, migrating glioblastoma cells accumulated extensively in the subpial zone of the cerebral cortex. These cells expressed both GluR1 and GluR4, but GluR2 (R) only faintly (Figs. 1d, e, f and g). In adjacent brain tissues (Figs. 1h, i, j, and k), invading undifferentiated tumor cells expressed GluR1 and GluR4 (Figs. 1i and k), whereas normal neurons expressed GluR2 (R) abundantly (Fig. 1j). Fusiform tumor cells invading the white matter expressed vimentin, a marker of immature astrocytes, and GluR1, but expressed very little GluR2 (R).

The present inventors prepared primary cultures of glioblastoma cells from surgical samples and examined the expression of AMPAR subunits (Table 1). In these cultures, most cells exhibited GFAP-immunoreactivity, indicating that they were of glial cell origin, and there was no contamination by neurons or microglial cells as judged by the lack of immunoreactivities for neurofilament protein (NFP) (Nature, 298, 277-279, 1982; Acta Neuropathol., 64, 30-36, 1984), a neuronal maker, and Ki-MlP (Pathol. Int., 46, 15-23, 1996), a marker of microglial cells, respectively.

In dual immunofluorescence using antibodies against GluR1 and GluR4, the majority of cultured cells expressed both proteins simultaneously (Figs. 1l, m and n). However, in dual immunofluorescence using antibodies against GluR1 and GluR2 (R) or antibodies against GluR4 and GluR2 (R), the only minority of GluR1- or GluR4-expressing cells showed GluR2 (R) immunoreactivities (Figs. 1o, p and q).

The present inventors established 4 glioblastoma cell lines from the surgical samples. Three of them expressed GluR1 plus GluR4, GluR1 plus GluR3, and GluR3 plus GluR4, respectively. One cell line expressed no AMPAR subunit. The present inventors also examined the expression of AMPAR subunits in a commercially available human glioblastoma cell line U87 MG. This cell line expressed predominantly GluR1 and GluR3, and GluR2 weakly (Table 1).

To confirm the expression of GluR1 ∼ 4 subunits in glioblastoma cells, the present inventors next conducted reverse-transcription polymerase-chain reaction (RT-PCR) studies (Neuron, 12, 383-388, 1994). A reverse transcription was performed on 3 surgical samples, 4 primary cultured cells and 5 cell lines, followed by PCR amplification with primers specific for GluRl - 4. The amplified products were then investigated by restriction analysis with enzymes specific for each GluR1 ∼ 4 fragments (Neuron, 12, 383-388, 1994). Fig. 1r shows a representative result obtained in a primary culture of tumor cells. In this case, expression of all GluRl - 4 mRNAs was detected. In control brain tissues adjacent to the tumor, expression of GluR1 ∼ 4 mRNA was also detected (Fig. 1s). Concerning the expression of GluRl - 4 mRNA, the results obtained by RT-PCR agreed completely with those obtained immunohistochemically (Table 1).

**(Table 1)**

| | Immunohistochemistry | | | | RT-PCR | | | |
|---|---|---|---|---|---|---|---|---|
| | R1 | R2 | R2/R3 | R4 | R1 | R2 | R3 | R4 |
| Surgical samples | | | | | | | | |
| GNS-3000 | +++ | + | + | ++ | | | | |
| GNS-3114 | ++ | ++ | ++ | +++ | + | + | + | + |
| GNS-3148 | ++ | +/- | +/- | ++ | | | | |
| GNS-3186 | ++ | + | + | ++ | | | | |
| GNS-3195 | ++ | + | + | ++ | | | | |
| GNS-3199 | ++ | + | + | ++ | | | | |
| GNS-3210 | +++ | + | + | +++ | | | | |
| GNS-3226 | ++ | + | + | ++ | | | | |
| GNS-3243 | ++ | ++ | ++ | +++ | | | | |
| GNS-3245 | ++ | + | + | ++ | + | + | + | + |
| GNS-3262 | ++ | + | + | ++ | | | | |
| GNS-3275 | ++ | + | + | ++ | | | | |
| GNS-3296 | ++ | + | + | ++ | | | | |
| GNS-3314 | +++ | + | + | +++ | + | + | + | + |
| G-22 | + | - | - | + | | | | |
| G-87535 | ++ | - | - | + | | | | |

| Cultured cells | | | | | | | | |
|---|---|---|---|---|---|---|---|---|
| GNS-3245 | +++ | - | + | + | + | - | + | + |
| GNS-3296 | ++ | + | + | ++ | + | + | + | + |
| GNS-3302 | +++ | + | + | +++ | + | + | + | + |
| GNS-3314 | +++ | ++ | ++ | +++ | + | + | + | + |

| Cell lines | | | | | | | | |
|---|---|---|---|---|---|---|---|---|
| CGNH-89 | ++ | - | - | ++ | + | - | - | + |
| CGNH-NM | ++ | - | + | - | + | - | + | - |
| MRCH-92 | - | - | + | + | - | - | + | + |
| TATE-87 | - | - | - | - | - | - | - | - |
| U87 MG | +++ | + | ++ | - | + | + | + | - |

These results indicated that AMPAR subunits are expressed in human glioblastoma cells, and that the GluR1 and GluR4 subunits are expressed more abundantly and consistently than the GluR2 (R) subunit. They strongly suggested that Ca²⁺-permeable AMPARs assembled without GluR2 (R) are formed in these cells.

### (Functional expression of Ca²⁺-permeable AMPARs in glioblastoma cells)

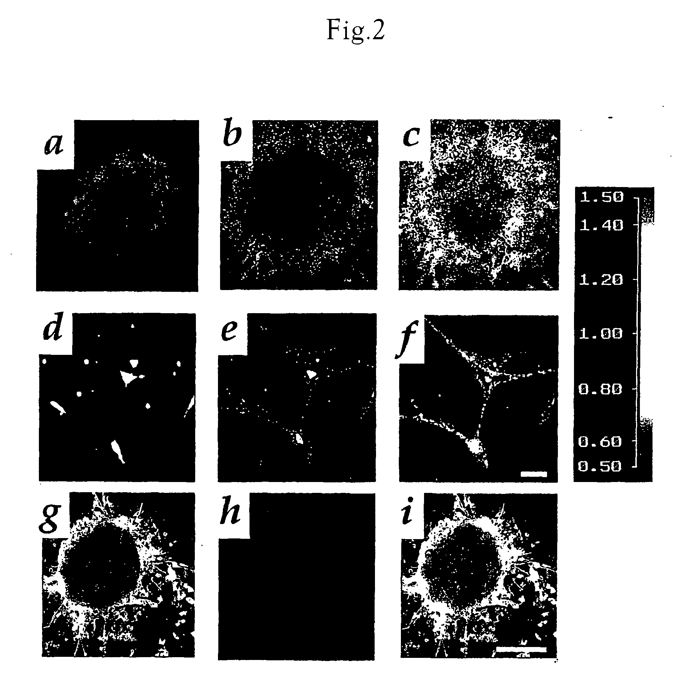

To examine functional expression of Ca²⁺-permeable AMPARs in glioblastoma cells in primary culture, the present inventors measured AMPA-induced changes in the intracellular [Ca²⁺]ᵢ (Ca²⁺ concentration), using fura-2 AM (See Fig. 2). Application of 200 µM AMPA together with 100 µM cyclothiazide (CTZ), which reduces desensitization of AMPARs, increased [Ca²⁺]ᵢ of the cultured cells (Figs. 2a, b and c). The increase in [Ca²⁺]ᵢ from 80 ± 21 to 285 ± 120 nM (n = 150) was detected in 65 ± 15% of cells in 16 different culture dishes. A prominent [Ca²⁺]ᵢ rise was occasionally seen in cells with long processes that were located in the periphery of the explant (Figs. 2d, e and f).

The increase in [Ca²⁺]ᵢ was selectively abolished by the AMPA antagonist, either 20 µM 2,3-dihydroxy-6-nitro-7-sulfamoyl-benzo(F)-quinoxaline (NBQX) or 10 µM [2,3-dioxo-7-(1H-imidazol-1-yl)-6-nitro-1,2,3,4-tetrahydroq uinoxalin-1-yl]acetic acid monohydrate (YM872) (J. Pharm. Pharmacol., 50, 795-801, 1998). In these cultured cells, 100 mM K⁺ did not increase [Ca²⁺]ᵢ, indicating that voltage-dependent Ca²⁺ channels were not involved in this [Ca²⁺]ᵢ increase. Expression of both vimentin and GluR1 was detected in the primary cultured cells, used for [Ca²⁺]ᵢ measurement (Figs. 2g, h and i).

### (Effects of adenoviral-mediated expression of AMPAR subunits)

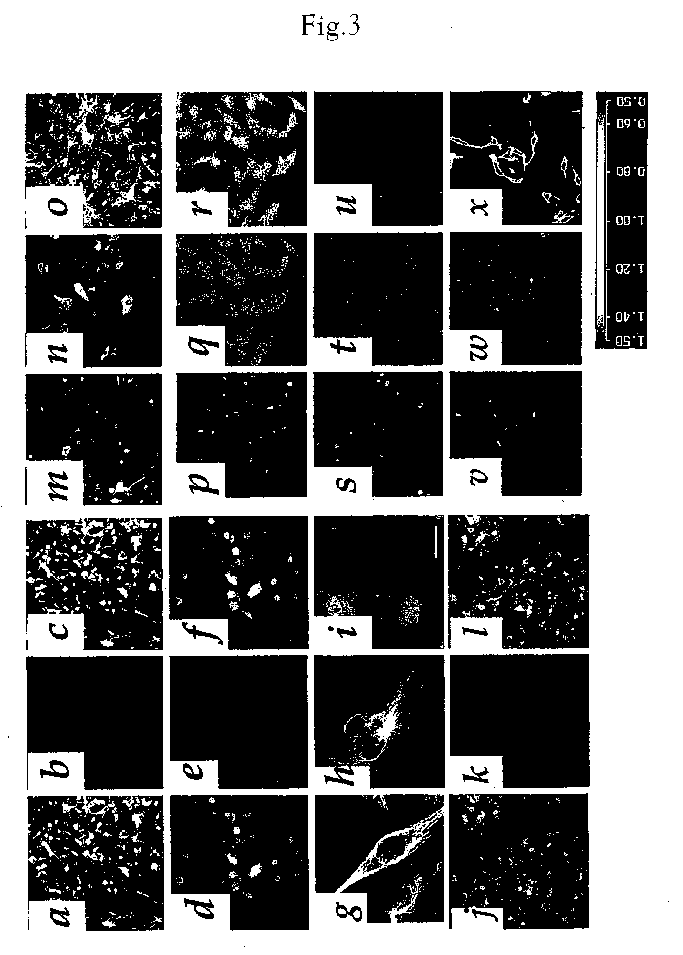

To elucidate the pathophysiological significance of Ca²⁺-permeable AMPARs in glioblastoma cells, the present inventors first attempted to convert Ca²⁺-permeable AMPARs into Ca²⁺-irmeable receptors by adenoviral-mediated transfer of GluR2 cDNA (Science, 292, 926-929, 2001; NeuroReport, 12, 745-748, 2001). The following experiments were performed mainly in an established glioblastoma cell line (Fig. 3), designated as CGNH-89, which possessed Ca²⁺-permeable AMPARs assembled from the GluR1 and/or GluR4 subunits.

To introduce the GluR2 (R) cDNA into the cultured cell, the present inventors constructed two recombinant adenoviruses; one was for expressing Cre recombinase (AxCANCre), and another was for expressing GluR2 (R), bearing a switching unit for regulating the expression of GluR2 (R) by Cre, consisting of the CAG promoter, a pair of loxP sequences, a stuffer gene and GluR2 (R) cDNA (AxCALNLGluR2). The present inventors also constructed a recombinant adenovirus bearing green fluorescent protein (GFP) cDNA (AxCAGFP), which was used as a marker of adenovirus-infected cells. In Fig. 3a, the cultured cells expressing GluR1 protein (Fig. 3b) were infected with AxCAGFP. GFP fluorescence was detected in most of cells expressing GluR1 2 days postinfection (Fig. 3c). When cells were infected with 3 kinds of recombinant adenoviruses, AxCANCre, AxCALNLGluR2 (R) and AxCAGFP, GluR2 (R) protein was detected in almost all cells emitting GFP fluorescence 2 days postinfection (Figs. 3d, e and f).

As a result, the present inventors noted a remarkable change in the morphology of cells expressing GluR2 (R) (Fig. 3d, e and f). The cell somata were gradually enlarged and flattened. The cytoplasmic processes were retracted within 4 days after viral infection. These characteristic changes were never seen when the cells were infected with AxCALNLGluR2 (R) and/or AxCAGFP, which induced no GluR2 (R) expression. The mean value of the surface area of the cell body was 360 ± 133 µm² (n = 30) in control cells infected with AxCALNLGluR2 (R) and/or AxCAGFP, whereas it was increased to 1230 ± 350 µm² (n ≈ 30) in cells expressing GluR2 (R) (P < 0.05).

The present inventors noted that the cytoskelton of GluR2 (R) expressing cells was disorganized (control cell in Fig. 3g vs. GluR2 (R)-expressing cell in Fig. 3h), and that the cells exhibited signs of apoptosis such as nuclear blebbing and the formation of apoptotic bodies (Fig. 3i). These characteristic changes never occurred when Ca²⁺-permeable AMPARs were overexpressed by introduction of either GluR1 or GluR2 (Q) cDNA. Instead, the present inventors noted a common feature in the cell cultures overexpressing Ca²⁺-permeable AMPARs in that enlarged fusiform cells often with long processes proliferated at high density (Figs. 3j, k and l).

The above changes induced in CGNH-89 cells by adenoviral-mediated expression of GluR2 (R) or GluR2 (Q) were also seen in U87 MG cells. The introduction of the GluR2 (R) gene made the somata of U87 MG cells enlarged and flattened, whereas that of the GluR2 (Q) gene increased the number of fusiform cells with long processes (Figs. 3m, n and o).

In Figs. 3p - x, the present inventors confirmed changes in Ca²⁺ permeability induced by adenoviral-mediated introduction of GluR2 (R) or GluR2 (Q) in CGNH-89 cells. Application of 200 µM AMPA together with 100 µM CTZ increased [Ca²⁺]ᵢ from 97 ± 21 to 275 ± 50 nM (n = 350) in 52 ± 14% of control cells in 10 culture dishes (Figs. 3p, q and r). The same treatment had no effect on [Ca²⁺]ᵢ in cells expressing GluR2 (Figs. 3s, t and u) in 4 culture dishes, whereas it caused a marked increase in [Ca²⁺]ᵢ from 77 ± 21 to 1185 ± 240 nM (n = 40) in cells expressing GluR2 (Q) in 2 culture dishes (Figs. 3v, w and x).

The present inventors examined whether CGNH-89 cells expressing GluR2 (R) could increase [Ca²⁺]ᵢ to a similar extent as control cells in response to ATP, which is known to mediate Ca²⁺ signaling in glial cells (J. Neurosci., 18, 8794-8804, 1998). The application of 25 µM ATP increased [Ca²⁺]ᵢ from 70 ± 24 to 1438 ± 174 nM (n = 50) in cells expressing GluR2 (R) in 5 culture dishes. This increase was almost similar to that detected in control cells, in which the same treatment increased [Ca²⁺]ᵢ from 77 t 28 to 1574 ± 161 nM (n = 50). This indicated that cells to which the GluR2 (R) gene was introduced were capable of responding to the external stimulus.

The present inventors next estimated the apoptotic index in CGNH-89 cells expressing GluR2 (R) using the terminal deoxynucleotide transferase (TdT)-mediated dUTP nick end labeling (TUNEL) method (Science, 267, 1445-1449, 1995), and compared this value with that in cells infected with only AxCALNLGluR2 (control). The apoptotic index was defined as the number of TUNEL-positive cells divided by the total number of propidium iodine (PI)-positive nuclei per microscopic field. To obtain the mean value in each culture dish, 10 to 15 randomly chosen fields were sampled. This index was 20.2 ± 4.3% in cells expressing GluR2 (R) (n = 4), which was significantly greater than that in controls (5.2 ± 1.2%, n = 4, P < 0.05).

The present inventors also examined proliferation activity of GluR2 (R)-expressing cells by calculating Ki-67 staining index (the number of Ki-67-positive cells divided by the total number of PI-positive nuclei per microscopic field (Lab. Invest., 70, 125-129, 1994). This index was 12.3 ± 2.3% in control and that of cells expressing GluR2 (R) was < 0.1% (n = 4, P < 0.001). In contrast, introduction of GluR2 (Q) cDNA (NeuroReport, 12, 745-748, 2001) for overexpression of Ca²⁺-permeable AMPARs significantly increased the Ki-67 staining index to 28.3 ± 4.8% (n = 4, P < 0.01), although no significant change in the apoptotic index was detected (4.3 ± 1.3%). When GluR1 was overexpressed by adenovirus-mediated transfer of the GluRl gene for overexpression of Ca²⁺-permeable AMPARs, the apoptotic index and the Ki-67 staining index were 5.3 ± 2.3% and 26.3 ± 4.5%, respectively. There were no significant changes in these 2 indices between cells expressing GluR2 (Q) and overexpressing GluR1.

The above results strongly suggested that blockage of Ca²⁺ influx through Ca²⁺-permeable AMPARs causes apoptotic changes in glioblastoma cells. The present inventors therefore examined effects of AMPAR antagonists on the cell shape, apoptotic and Ki-67 staining indices in CGNH-89 cells. The application of 20 µM NBQX or 10 µM YM872 for 2 days caused changes in the morphology of cells, namely, retraction of cytoplasmic processes, enlarged and flattened somata, similar to those seen following GluR2 (R) expression. Furthermore, NBQX and YM872 significantly increased the apoptotic index from 3.0 ± 1.8% to 13.0 ± 2.0% (n = 4, P < 0.01), and from 3.8 ± 2.3% to 18.0 ± 2.5% (n = 4, P < 0.01), respectively. They markedly decreased the Ki-67 staining index from 18.5 ± 3.0% to 2.0 ± 1.0% (n = 4, P < 0.01), and from 16.4 ± 3.5% to < 0.1% (n = 4, P < 0.01), respectively.

In the other 3 cell lines expressing Ca²⁺-permeable AMPARs (CGNH-NM, MRCH-92 and U87 MG in Table 1), both GluR2 (R) expression and application of NBQX or YM872 caused similar morphological changes in cultured cells, increased the apoptotic index, and decreased the Ki-67 staining index. The application of these AMPAR antagonists to cells to which the GluR2 (R) gene was introduced caused no further changes in cell shapes and these 2 indices in all 4 cell lines tested, indicating that the viral-mediated expression of GluR2 (R) and the AMPAR antagonists act on the same target.

### (Promotion of cell migration by Ca²⁺-permeable AMPARs)

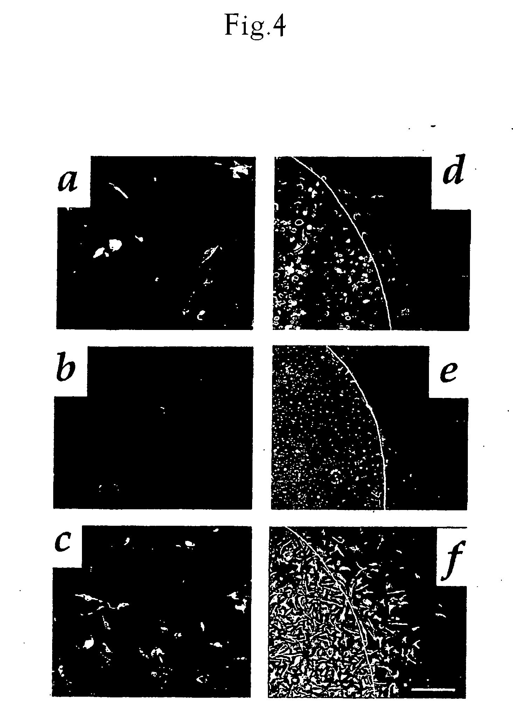

The migratory tumor cells in the white matter in the brain exhibited fusiform morphology and often had long cellular processes. These shapes of tumor cells seemed to be suitable for cell migration. In fact, cell migration was markedly inhibited when the expression of GluR2 (R) in the tumor cells caused them to adopt a flattened shape and retracted their processes. In contrast, overexpression of Ca²⁺-permeable AMPARs by adenoviral-mediated introduction of GluR2 (Q) elongated cellular processes and appeared to promote their migratory behavior. To estimate the degree of cell migration quantitatively, the present inventors conducted 2 experiments (Fig. 4).

First, the present inventors used a transwell double chamber culture dish to estimate motile activity in the direction of the Z-axis. This culture dish had the top and bottom chambers separated by a porous membrane with mult iple pores 8 µm in diameter. When cells in the top chamber were infected with AxCAGFP and AxCALNLGluR2 (R) without AxCANCre (control), a small portion of cells moved into the bottom through the porous membrane within 24 hr. The mean value of migrated cells counted in ten 20 × objective fields under the microscope was 4.2 ± 2.3 in 5 independent experiments (Fig. 4a). However, no viable cell expressing GluR2 (R) moved down, and only fragments of the disrupted cells were seen in the bottom chamber (Fig. 4b). In contrast, the introduction of GluR2 (Q) increased the number of cells that moved into the bottom chamber (31.2 ± 8.3, n = 5, P < 0.02) (Fig. 4c).

The present inventors next examined motile activity using a cloning ring (7 mm in diameter) in the center of the culture dish. Cells were cultured inside the ring that was removed 48 hr. after viral infection. Then, the number of cells that crossed the border of the cloning ring during the next 24 hr. was counted. The mean number of cells in 5 experiments were 43.2 ± 5.3, 15.0 ± 3.3 and 250.2 ± 50.3 in cells expressing GFP, GluR2 (R), and GluR2 (Q), respectively (Figs. 4d, e and f). Thus, the expression of GluR2 (R) significantly suppressed the cell motility (P < 0.002), whereas that of GluR2 (Q) promoted it (P < 0.01).

Similar inhibitory effect of tumor migration was observed by application of 10 µM YM872. The introduction of GluR2 (R) gene and application of YM872 also inhibited the cell migration in the other 3 cell lines expressing Ca²⁺-permeable AMPARs. (Suppression of tumor invasion by GluR2 (R) expression and AMPA antagonist in the nude mouse model of human glioblastoma)

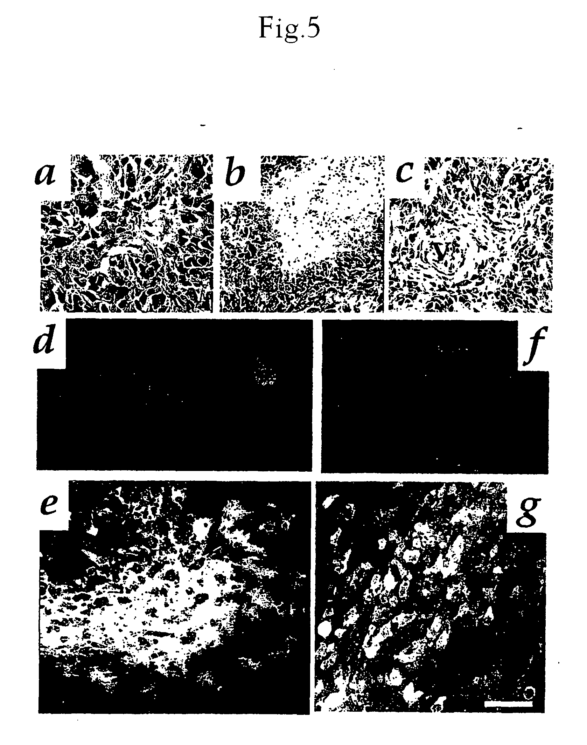

The present inventors finally examined whether GluR2 (R) expression affected migration and proliferation of the glioblastoma in vivo (Fig. 5). For this purpose, the present inventors first used a mouse model of human glioblastoma in which 2 × 10⁵ CGNH-89 cells tagged with GFP using AxCAGFP were grafted into the cerebral subcortex of nude mice. These grafted cells formed cell clusters, invaded the myelinated pathway of the corpus callosum as seen in human surgical samples, and spread widely throughout the brain in all 15 nude mice tested. The histology of the nude mouse xenograft samples showed characteristic features of human glioblastoma; that is, pleomorphism (Fig. 5a), necrosis with pseudopalisading (Fig. 5b) and microvascular proliferation (Fig. 5c).

Fig. 5b shows a cluster of tumor cells formed in the subcortical area 9 days after grafting of 2 × 10⁵ cells infected with AxCAGFP and AxCALNLGluR2 (R) for expression of only GFP. The tumor cells, the cell nuclei of which were stained with PI, were densely packed, and GFP was detected in cells in the inoculated region (Fig. 5e). In contrast, when the same number of cells were inoculated after they had been infected with AxCALNLGluR2 (R) plus AxCANCre together with AxCAGFP for expression of both GluR2 (R) and GFP, no solid cluster of tumor cells was formed in any of 13 nude mice tested (Fig. 5f). The tumor cells ceased to proliferate and became apoptotic as judged by nuclear morphology after PI staining (Fig. 5g).

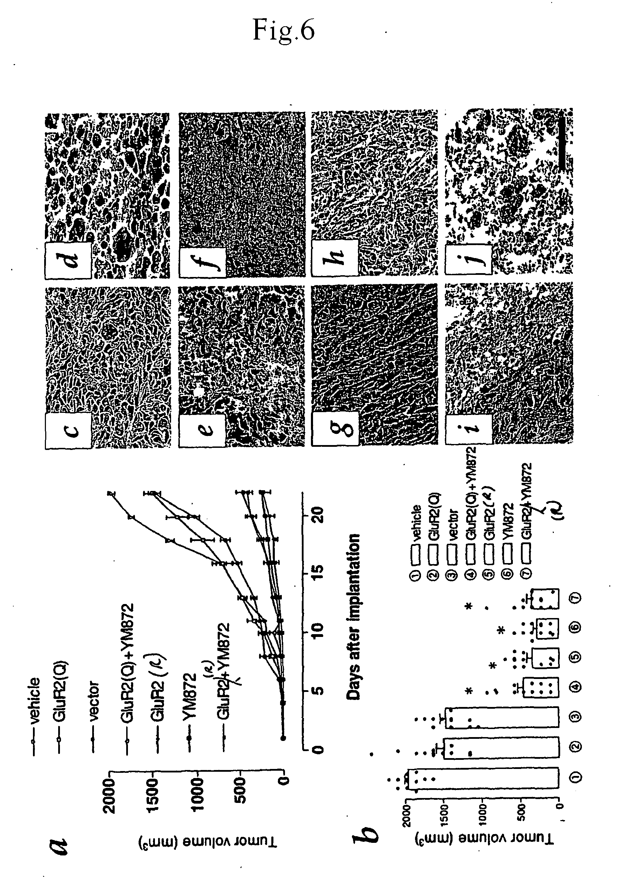

To further estimate quantitatively whether manipulation of Ca²⁺-permeable AMPARs alters tumor growth in vivo, the present inventors examined effects of GluR2 (R) and GluR2 (Q) expression and/or application of the AMPAR antagonist YM872 on the growth of tumor produced by grafting CGNH-89 cells into the subcutaneous tissue of nude mice (Fig. 6) - (Figs. 6a and b). The adenoviral-mediated expression of GluR2 (R) significantly reduced the rate of tumor growth as well as the size of tumor 22 days after grafting, compared to the case where tumor was infected with AxCALNLGluR2 (R) without AxCANCre (n = 12, P < 0.001). The expression of GluR2 (Q) tended to accelerate the tumor growth, although it did not increase the size of tumor 22 days after grafting. Intraperitoneal application of YM872 significantly reduced the size of tumor, compared to the treatment with the vehicle (phosphate buffered saline; PBS) (n = 12, P < 0.001). It also inhibited the growth of tumor expressing GluR2 (Q) (n = 12, P < 0.001), but had no further inhibitory effects on tumor expressing GluR2 (R).

Histological analysis revealed the presence of numerous mitotic cells in tumor tissues treated with the vehicle alone (Fig. 6c). In tumor tissues treated with YM872 (Fig. 6d) or expressing GluR2 (R) (Figs. 6e and f), mitotic cells were scarcely detected. Instead, a large number of apoptotic cells were observed. Proliferation of tumor cells expressing GluR2 (Q) was inhibited by application of YM872 (Figs. 6g, h and i). Treatment with YM872 caused no further inhibition of tumor proliferation in cells expressing GluR2 (R) (Fig. 6j).

### [Consideration of experiments]

In the surgical samples obtained from glioblastoma patients, migratory cells in the myelinated pathway exhibited fusiform or bipolar morphologies and expressed GluR1 and/or GluR4 proteins abundantly. The migrating cells located in the periphery of explant cultures derived from the surgical samples also had fusiform morphology often with long cellular processes, and expressed GluR1. Overexpression of Ca²⁺-permeable AMPARs by adenoviral-mediated transfer of GluR1 or GluR2 (Q) increased the number of migratory cells with fusiform morphology with long cellular processes. In contrast, the motility of tumor cells was markedly suppressed after the cellular morphology had been transformed to a polygonal and flattened shape by GluR2 (R) expression. It seems likely that the fusiform morphology and elongatedprocesses wouldbe suitable for cellmigration, because the fusiform morphology would make it possible for the tumor cells to invade the compact myelinated pathways or the narrow space in the CNS tissues.

The elongation of cellular processes is considered to be the first step for cell migration, which is followed by translocation of cell soma and retraction of rear processes (Cell, 84, 371-379, 1996). With regard to the causal relationship between the morphology of glial cells and Ca²⁺-permeable AMPARs, the present inventors have previously shown that GluR2 (R) expression causes marked retraction of glial processes, whereas overexpression of GluR2 (Q) causes their elongation in Bergmann glia-like cultured cells derived from the mouse cerebellum (NeuroReport, 12, 745-748, 2001). However, detailed mechanism responsible for elongation of glial processes mediated by Ca²⁺-permeable AMPARs remains to be elucidated.

In this experiment, the present inventors showed that the conversion of Ca²⁺-permeable AMPARs to Ca²⁺-impermeable receptors increased the apoptotic index and suppressed the proliferation activity of tumor cells. Thus, Ca²⁺ influx through these receptors seems to be required for stimulation of the anti-apoptotic signaling cascade. This notion is supported by the previous finding that a modest amount of Ca²⁺ influx is indispensable for the survival of several cultured cells such as cerebellar granule cells and NB108 neuroblastoma cells (J. Neurosci., 7, 2203-2213, 1987; Proc. Natl. Acad. Sci. USA, 86, 6421-6425, 1989; Nature, 396, 584-587, 1998). Yano et al. (Nature, 396, 584-587, 1998) have demonstrated that in NB108 neuroblastoma cells the Ca²⁺ supplied by Ca²⁺ entry through N-methyl-D-aspartate (NMDA) receptors activates Ca²⁺/calmodulin-dependent protein kinase kinase (CaM-KK), which in turn phosphorylates a serine/threonine kinase, Akt (protein kinase B). The activation of Akt is known to release a variety of anti-apoptotic signals, thus facilitating cell survival (Nature, 396, 584-587, 1998).

The endogenous agonist for AMPARs is glutamate. The culture medium in this experiment was supplemented with fetal calf serum (FCS) and contained up to 100 µM glutamate. The presence of glutamate was essential for maintenance of fusiform morphology as well as proliferation of the tumor cells. When the cells were cultured in glutamate-free medium supplemented with 10% dialyzed FCS for 4 ∼ 5 days, they became polygonal in shape and the number of apoptotic cells increased. These changes were restored by addition of 100 µM glutamate. An obvious question is whether a sufficient concentration of glutamate to activate Ca²⁺-permeable AMPARs is actually supplied to proliferating and migrating tumor cells in the brain. It has been shown that implanted glioma cells continue to secrete glutamate, resulting in a detectable elevation of extracellular glutamate, and that gliomas with high glutamate release have a distinct growth advantage (Nature Med., 7, 1010-1015, 2001). It is likely that the glioblastoma cells secrete glutamate in an autocrine manner similarly as astrocytes do various cytokines and growth factors (Nature, 391, 281-285, 1998; J. Neuropathol. Exp. Neurol., 57, 653-663, 1998).

Both NMDA and AMPA receptor antagonists have been shown to inhibit proliferation and migration of various types of tumor cells including glioma and neuroblastoma cells (Nature Med., 7, 1010-1015, 2001; Proc. Natl. Acad. Sci. USA, 98, 6372-6377, 2001). In this study, the present inventors showed that Ca²⁺-permeable AMPARs play critical roles for facilitating migration and proliferation of the human glioblastoma cells, and that the blockage of Ca²⁺-permeable AMPARs by GluR2 (R) expression or YM872 effectively suppresses tumor growth. Thus, Ca²⁺-permeable glutamate receptors may be a therapeutic target for treatment of brain tumors.

### [Method used in experiment]

### (Surgical samples and cell cultures)

Sixteen surgical samples examined in this experiment were histologically diagnosed as glioblastoma multiforme according to WHO classification. Cell cultures were prepared as described previously (Acta Neuropathol., 94, 425-435, 1997; J. Neurosci. Res., 51, 526-535, 1998). Among 4 glioblastoma cell lines, CGNH-89 exhibited high tumorigenicity and was used for heterotransplantation into nude mice. Cells were cultivated in Dulbecco' s modified Eagle's medium (DMEM) (Life Technologies) supplemented with 10% FCS and 2 mM glutamate. The concentration of glutamate in this medium determined by enzymatic cycling was 107 ± 5 µM (n = 4) (Brain Res., 573, 197-203, 1992).

### (Gene transfer using adenoviral vectors)

The present inventors constructed the following recombinant adenoviruses (Proc. Natl. Acad. Sci. USA, 93, 1320-1324, 1996; Nucl. Acids Res., 23, 3816-3821, 1995).
(1) AxCALNLGluR1, AxCALNLGluR2 (R) and AxCALNLGluR2 (Q): expression-switchingunits for expression of the rat GluR1, GluR2 (R) and GluR2 (Q) subunits, respectively, consisting of the CAG promoter, a loxP sequence, a stuffer gene (neo-resistance gene), a poly (A) signal, a second loxP site, the GluR1, GluR2 (R) or GluR2 (Q) coding sequence, and another poly (A) signal (Mol. Brain Res., 65, 176-185, 1999). Rat GluR1 and GluR2 cDNAs were kind gifts from Drs. S. Heinemann and M. Hollmann.
(2) AxCANCre and AxCAGFP: recombinant adenoviruses for expression of Cre recombinase and EGFP (Clontech), respectively. Cells were infected at a multiplicity of infection (MOI) of 5 with each of the recombinant adenoviruses 2 - 5 days before experiments.

### (Histology)

Immunohistochemical staining was performed with selective antibodies against GluR1, GluR2 GluR2/GluR3, GluR4 (Chemicon), GFAP (Virchows Arch. A Pathol. Anat. Histopathol., 405, 299-310, 1985) andvimentin (V9, Dakopatts A/S) as described previously (NeuroReport, 12, 745-748, 2001; ActaNeuropathol., 94, 425-435, 1997). Immunoreactivities were visualized with diaminobenzidine. For dual immunofluorescence, FITC-, rhodamine- and Alexa 594-labeled secondary antibodies (Molecular Probe) were used to visualize the bound antibodies. The stained cells were viewed with a laser-scanning confocal microscope (MRC-1024E, Bio-Rad). In situ hybridization was conducted with the same method as described by Kondo et al (J. Neurosci., 17, 1570-1581, 1997).

### (Reverse transcription-polymerase chain reaction (RT-PCR))

Aliquots of 100 ng of RNA prepared from samples were reverse-transcribed with random hexamers (Roche Diagnostics) and the RT product was subjected to 35 PCR cycles (denaturation for 20 sec. at 94°C, annealing for 20 sec. at 50°C, extension for 20 sec. at 72° C), with primers that simultaneously amplified GluR1 ∼ GluR 4 (Neuron, 12, 383-388, 1994) (sense primer: 5'-CCTTTGGCCTATGAGATCTGGATGTG-3' (SEQ ID No. 1), antisense primer: 5'-TCGTACCACCATTTGTTTTTCA-3' (SEQ ID No. 2)).

A second PCR (35 cycles) for subunit specific amplification was performed with the sense primers specific for either GluR1 (base sequence at 1712 - 1733: 5'-AAGAGGGACGAGACCAGACAAC-3' (position 1 being the first nucleotide of the coding sequence; SEQ ID No. 3)), GluR2 (R) (base sequence at 1732 - 1755: 5'-GAAGATGGAAGAGAAACACAAAGT-3' (SEQ ID No. 4)), GluR3 (base sequence at 1749 - 1771: 5'-GGAAGACAACAATGAAGAACCTC-3' (SEQ ID No. 5)), or GluR4 (base sequence at 1747 - 1766: 5'-GAAGGACCCAGCGACCAGCC-3' (SEQ ID No. 6)) and the common antisense primer used for the first amplification. The products of this second amplification (637 bp for GluR1, 638 bp for GluR2, 657 bp for GluR3 and 626 bp for GluR4) were digested by restriction enzymes specific for GluR1 (Bgl I), GluR2 (R) (Bsp1286 I), GluR3 (Ava I) and GluR4 (Pvu II).

### (Intracellular Ca²⁺ imaging)

Cells were loaded with fura 2-AM (Dojin) at room temperature for 40 min. Fluorescence images were obtained at an excitation wavelength of 340 and 380 nm using the Argus/HiSCA system (Hamamatsu Photonics). The 340/380 nm fluorescence ratio was converted to [Ca²⁺]ᵢ as described previously (Cell, 88, 49-55, 1997).

### (TUNEL, proliferation and migration assays)

For TUNEL and cell proliferation assays, cells were plated at a density of 2 × 10⁴/well in Lab-Tek 4-well glass slides filled with the standard culture medium. At 6 hr. after plating, the medium was replaced with glutamate-free medium and cells were infected with the adenoviruses. At 48 hr. after plating, glutamate (100 pM) was added to the medium and cells were fixed at 5 days postinfection. TUNEL assay was performed with an In Situ Cell Death Detection kit (MBL). Cell proliferation assay was performed with anti-Ki-67 monoclonal antibody (Dako) staining indices. Migration assay was performed in a transwell chamber (8 µm pore size) (Corning Corstar Corp.).

Cells were plated at a density of 5 × 10⁴/well in the top chamber. At 6 hr. after plating, the cells were infected with the recombinant adenoviruses in glutamate-free medium supplemented with 10% dialyzed FCS. At 48 hr. postinfection, glutamate (100 pM) was added to the medium. Cells were cultivated for further 24 hr., and the number of cells that migrated through porous membrane was counted with a 20 × objective under the microscope.

In another set of migration assay, a glass cloning cylinder (7 mm in diameter) coated on one end with silicone grease was placed in the center of the culture dish. Within the cylinder 2 × 10⁴ cells were plated. At 48 hr. postinfection, the cloning ring was removed and cells were cultivated for further 24 hr. Then, the number of cells that crossed the border of the cloning ring was counted.

### (Animal models)

CGNH-89 cells were suspended at 1 × 10⁷/100 µl in culture medium. Aliquots of 2 µl of the suspension infected with AxCAGFP and AxCALNLGluR2 (R) with or without AxCANCre were injected stereotaxically into the brains of nude mice under ether anesthesia. Brain tissues were examined 9 to 14 days postinjection.

In addition, the effect of adenoviral-mediated expression of GluR2 (R) and GluR2 (Q) on tumor growth was evaluated quantitatively in subcutaneous tumors with or without application of YM872, an antagonist for AMPA-type glutamate receptors. Cell suspensions (1 × 10⁷/100 pl) were injected subcutaneously in the flank of nude mice 5 ∼ 6 weeks old (body weight, 18 - 20 g). Adenoviruses (1 × 10⁷ PFU, diluted in a total of 100 µl of PBS) were administered intratumorally once 5 days after tumor inoculation with a 27-gauge needle. AxCALNLGluR2 (R) without AxCANCre was administered for control.

YM872, a kind gift from Yamanouchi Pharmaceutical Company, was dissolved in PBS. Dose-dependent inhibition of tumor growth was observed after daily intraperitoneal injection of YM872 (starting one day after tumor implantation) at doses of 10, 50 and 100 mg/kg for 2 weeks. In this series of experiments, 100 mg/kg YM872 was used. PBS (100 pl) was administered intraperitoneally for control. Tumor volume was calculated according to the formula (length × width²)/2. At the end of each experiment, tumor tissues were subjected to histological analysis.

### (Data analysis)

Data are expressed as means ± s. e. m. Statistical comparisons were performed by unpairedt-test or one-way analyses of variance (ANOVAs: Scheffe's test for post-hoc comparison).

### [Figure legends]

(Fig. 1 A set of photographs showing AMPA receptors expressed in human glioblastoma cells)
   a ∼ c; Serial sections from an original surgical sample. a; Subpial accumulation of invading tumor cells stained with hematoxylin-eosin (HE). b; Expression of GluR1 mRNA detected by in situ hybridization (nitro blue tetrazolium chloride, blue). c; Image of immunofluorescence for GluR1 (rhodamine, red) is shown.
   d ∼ g; Serial sections from an original surgical sample (GNS-3148). d; Subpial accumulation of invading tumor cells (HE staining) is shown. e; Immunostaining with anti-GluR1 antibody. f; Immunostaining with anti-GluR2 antibody. g; Immunostaining with anti-GluR4 antibody.
   h ∼ k; An adjacent tissue from the same samples of "d" ∼ "g" . h; Invading tumor cells in the cerebral cortex (HE staining). i, j and k; Immunostainings with anti-GluRl, anti-GluR2 and anti-GluR4 antibodies, respectively.
   1 - n; Immunostaining with anti-GluR1 antibody (Alexa 594, red) (l) and anti-GluR4 antibody (FITC, green) (m), and their merged image (n) in tumor cells in primary culture are shown.
   o ∼ q; Immunostaining with anti-GluR1 antibody (Alexa 594, red) (o) and anti-GluR2 antibody (FITC, green) (p), and their merged image (q) in tumor cells are shown. Scale bar in "q" represents 100 µm for "a" ∼ "g", and 50 µm for "h" ∼ "q".
   r ∼ s; RT-PCR analysis with specific primers for GluR1 ∼ 4 in tumor cells in primary culture (r) and in normal brain tissue adjacent to the tumor before resection (s). In both "r" and "s", the left and right 4 lanes (R1 ∼ R4) show electrophoresis of PCR products before and after digestion by restriction enzymes specific for each GluR1 ∼ 4 DNA, respectively. The size of digested fragments on the right lanes is: 189 and 448 bp for GluR1 cut by Bgl I, 368 and 270 bp for GluR2 cut by Bsp1286 I, 420 and 237 bp for GluR3 cut by Ava I, 379 and 247 bp for GluR4 cut by Pvu II. Lane φ shows DNA size marker with the dense 500 bp band.
(Fig. 2 A set of photographs showing AMPA-induced changes in [Ca²⁺]ᵢ in cultured tumor cells)
   a ∼ c; AMPA-induced increase in [Ca²⁺]ᵢ in tumor cells in primary culture loaded with fura-2 AM is shown. Fluorescence image at the excitation wavelength of 340 nm (a) and pseudocolor images of the 340/380 nm ratio before (b) and during (c) exposure to 200 µM AMPA and 100 µM CTZ are shown.
   d ∼ f; AMPA-induced increase in [Ca²⁺]ᵢ in cells in monolayer that moved out from the explant culture is shown. The images were taken in the same arrangement as in "a" ∼ "c".
   g ∼ i; Dual immunofluorescence for vimentin (g, FITC, green), and GluR1 (h, rhodamine, red) and their merged image (i) are shown. Scale bar in "f" represents 20 µm for "d" ∼ "f" and that in "i" 100 µm for "a" ∼ "c" and "g" ∼ "i".
(Fig. 3 A set of photographs showing the effects of adenoviral-mediated expression of AMPA receptor subunits)
   a ∼ c; Control. Cultured cells (CGNH-89) were infected with AxCAGFP and AxCALNLGluR2 (R) without AxCANCre. GFP fluorescence (a), immunofluorescence for GluR1 (b) and their merged image (c).
   d ∼ f; Cells expressing GluR2. They were infected with AxCAGFP and AxCALNLGluR2 (R) together with AxCANCre. GFP fluorescence (d), immunofluorescence for GluR2 (e) and their merged image (f).
   g; Cells infected with AxCALNLGluR2 (R) alone for control, and dually stained for vimentin (FITC, green) and propidium iodine (PI) (red).
   h; Cells infected with AxCALNLGluR2 (R) together with AxCANCre, and dually stained for vimentin (FITC, green) and GluR2 (rhodamine, red). Note disrupted vimentin filaments in a cell expressing GluR2 (R) protein.
   i; Cells infected with AxCAGFP and AxCALNLGluR2 (R) together with AxCANCre, and stained with PI. Note apoptotic bodies and nuclear blebbing.
   j ∼ l; Cells overexpressing GluRl. They were infected with AxCALNLGluR1 together with AxCANCre. Immunofluorescence for vimentin (j, FITC, green) and GluR1 (k, rhodamine, red) and their merged image (1) are shown.
   m ∼ o, U87-MG cells infected with AxCAGFP (m), AxCAGFP and AxCALNLGluR2 (R) together with AxCANCre (n), and AxCAGFP and AxCALNLGluR2 (Q) together with AxCANCre (o). They were stained for GluR2 (R) (rhodamine, red). Merged images of GFP and GluR2 immunofluorescence are shown. Scale bar in "i" represents 100 µm for "a" ∼ "f" and "j" ∼ "o", and 20 µm for "g" ∼ "i".
   p ∼ x; Results of [Ca²⁺]ᵢ measurement using fura-2AM are shown. Control CGNH-89 cells (p - r) and cells expressing GluR2 (R) (s - u) and GluR2 (Q) (v - x). From left to right, fluorescence images at the excitation wavelength of 340 nm, pseudocolor images of the 340/380 nm ratio before and during exposure to 200 µM AMPA and 100 µM CTZ.
(Fig. 4 A set of photographs showing the effects of expression of GluR2 (R) and GluR2 (Q) on cell migration)
   a - c; A set of views showing motility of tumor cells examined with a transwell double chamber. Confocal microscopic views of cells in the bottom chamber under the porous membrane. Cells were stained with anti-GluR2 (rhodamine, red) and anti-vimentin antibodies (FITC, green). a; Cells infected with AxCALNLGluR2 (R) without AxCANCre. Some cells expressing vimentin migrated across the porous membrane during 24 hr. b; Cells infected with AxCANCre and AxCALNLGluR2 (R) for expression of GluR2 (R). Only fragments of the disrupted cells were seen in the bottom chamber after 24 hr. c; Cells infected with AxCANCre and AxCALNLGluR2 (Q) for expression of GluR2 (Q). A larger number of tumor cells than in "a" migrated across the porous membrane during 24 hr.
   d ∼ f; Motility of tumor cells examined with a cloning ring is shown. Migration of cells infected with AxCALNLGluR2 (R) alone (d, control), GluR2 (R)-expressing cells (e) and GluR2 (Q)-expressing cells (f) within 24 hr. after removal of the cloning ring are shown. Cells were stained with anti-vimentin and anti-GluR2 antibodies. White lines indicate the border of the cloning ring. Scale bar in "f" represents 50 µm for "a" - "c" and 100 µm for "d" ∼ "f".
(Fig. 5 A set of photographs showing the effects of GluR2 (R) expression on tumor transplantation)
   a - c; Histopathology of the tumor formed by transplantation of cultured glioblastoma cells into the subcutaneous of the nude mouse is shown. The tumor was characterized by pleomorphism (a), necrosis with pseudopalisading (b), and microvascular proliferation (c). In the figure, 'v' indicates tumor vessels (HE staining).
   d; Tumor formation at 9 days after transplantation of 2 × 10⁵ cultured glioblastoma cells into the subcortical area of the nude mouse cerebrum is shown. The cultured cells had been infected with AxCAGFP and AxCALNLGluR2 (R) each at MOI 5 for expression of GFP (green) 2 days before transplantation, and stained by PI (red). e; Higher magnification view of the boxed area in "d".
   f ; Cells at 14 days after transplantation of 2 × 10⁵ cultured glioblastoma cells. The cultured cells had been infected with AxCAGFP and AxCALNLGluR2 (R) together with AxCANCre each at MOI 5 for expression of both GFP and GluR2 (R) 2 days before transplantation.
   g; Higher magnification view of the boxed area in "f". Note apoptotic nuclear morphology caused by expression of GluR2 (R). Scale bar in "g" represents 50 µm for "a", "e" and "g", 100 µm for "b", 75 µm for "c", 1500 µm for "d", and 1000 µm for "f".
(Fig. 6 A set of views showing the effects of manipulation of AMPA receptors on tumor growth)
   a; Effects of various treatments on growth rate of tumors grafted into the subcutaneous tissue of nude mice: injection of PBS (vehicle as control for application of YM872, inverted triangles); expression of GluR2 (Q) (open squares); injection of AxCALNLGluR2 without AxCANCre (vector as control for expression of GluR2 and GluR2 (Q), filled circles); expression of GluR2 (Q) plus application of YM872 (open triangles); expression of GluR2 (R) (filled diamonds); application of YM872 (filled squares); and expression of GluR2 (R) plus application of YM872 (filled triangles). For each treatment, 12 animals were used. Each plot represents the mean ± s. e. m. (n = 12) of tumor volume.
   b; Plots of tumor volumes measured 22 days after inoculation are shown. For each treatment, raw data obtained from 12 animals are plotted. * indicates significant difference at p < 0.001 relative to control (either vehicle or vector).
   c ∼ f; Histology of tumor tissues treated with the vehicle (c) and YM872 (d), and of those to which the GluR2 (R) gene was delivered (e, f) are shown. c, d and e; HE staining. f; Immunostaining with anti-GluR2 antibody.
   g ∼ h; HE staining (g) and immunostaining with anti-GluR2 antibody (h) in tumor tissues to which the GluR2 (Q) gene was delivered.
   i; HE staining of tumor tissues expressing GluR2 (Q) and treated with YM872 is shown.
   j; HE staining of tumor tissues expressing GluR2 (R) and treated with YM872 is shown. The tissues in "c" ∼ "j" were taken 22 days after tumor inoculation. Scale bar in "j" represents 50 µm for 'c" ∼ "j".

### Industrial Applicability

The present invention makes it possible to inhibit proliferation and invasion of brain tumor cells by regulating Ca²⁺ permeability by AMPA-type glutamate receptor subunits in developing animal brain tumor cells. In particular, the present invention makes it possible to regulate Ca²⁺ permeability in brain tumor cells by introducing a gene of an AMPA-type glutamate receptor subunit GluR2 (R) into a brain tumor cell, and to inhibit proliferation and invasion of brain tumor cells, and therefore, the present invention serves as effective means for treating brain tumors such as glioblastoma, which is known as the most malignant and highly migrative and invasive tumor. In addition, as the present invention makes it possible to measure proliferation/invasion activity of brain tumor cells by detecting/measuring the expression of AMPA-type glutamate receptor subunits in developing animal brain tumor cells, grade of malignancy of brain tumor can be found by using the method, and the present invention serves as powerful means for diagnosis, prevention or treatment of brain tumors.

## Claims

1. Use of a gene encoding an AMPA-type glutamate receptor subunit GluR2 (R) for the manufacture of a medicament for inhibiting the in vivo proliferation and invasion of brain tumor cells by regulating Ca²⁺ permeability by an AMPA-type glutamate receptor subunit GluR2 (R) being expressed in a developing animal brain tumor cell, wherein the gene used in the manufacture of the medicament encodes GluR2(R) without further RNA editing.

2. The use according to claim 1, wherein the gene encoding an AMPA-type glutamate receptor subunit GluR2 (R) is introduced into a developing animal brain tumor cell and expressed.

3. The use according to claim 1 or 2, wherein the gene encoding an AMPA-type glutamate receptor subunit GluR2 (R) is a cDNA of an AMPA-type glutamate receptor subunit GluR2 (R).

4. The use according to any one of claims 1 to 3, wherein the gene encoding an AMPA-type glutamate receptor subunit GluR2 (R) is introduced into a developing animal brain tumor cell by an expression vector, and is expressed.

5. The use according to claim 4, wherein the expression vector is a vector using an adenovirus.

6. The use according to any one of claims 1 to 5, wherein the brain tumor cell is a glioblastoma.

7. A method for measuring proliferation/invasion activity of brain tumor cells wherein the expression of an AMPA-type glutamate receptor subunit GluR2 (R) in a developing animal brain tumor cell is detected/measured.

8. A gene encoding an AMPA-type glutamate receptor subunit GluR2 (R) for use in inhibiting the in vivo proliferation and invasion of brain tumor cells by regulating Ca²⁺ permeability by an AMPA-type glutamate receptor subunit GluR2 (R) being expressed in a developing animal brain tumor cell, wherein the gene encodes GluR2(R) without further RNA editing.

9. The gene for the use according to claim 8, wherein the gene encoding an AMPA-type glutamate receptor subunit GluR2 (R) is introduced into a developing animal brain tumor cell and expressed.

10. The gene for the use according to claim 8 or 9, wherein the gene encoding an AMPA-type glutamate receptor subunit GluR2 (R) is a cDNA of an AMPA-type glutamate receptor subunit GluR2 (R).

11. The gene for the use according to any one of claims 8 to 10, wherein the gene encoding an AMPA-type glutamate receptor subunit GluR2 (R) is introduced into a developing animal brain tumor cell by an expression vector, and is expressed.

12. The gene for the use according to claim 11, wherein the expression vector is a vector using an adenovirus.

13. The gene for the use according to any one of claims 8 to 12, wherein the brain tumor cell is a glioblastoma.

## Patentansprüche

1. Verwendung eines Gens, welches eine AMPA-Typ-Glutamatrezeptor-Untereinheit GluR2(R) codiert, für die Herstellung eines Medikaments zum Hemmen der in vivo-Proliferation und -Invasion von Himtumorzellen durch Regulieren der Ca²⁺-Permeabilität durch eine AMPA-Typ-Glutamatrezeptor-Untereinheit GluR2(R), die in einer sich entwickelnden tierischen Hirntumorzelle exprimiert wird, wobei das bei der Herstellung des Medikaments verwendete Gen GluR2(R) ohne weiteres RNA-Editing codiert.

2. Verwendung nach Anspruch 1, wobei das Gen, welches eine AMPA-Typ-Glutamatrezeptor-Untereinheit GluR2(R) codiert, in eine sich entwickelnde tierische Hirntumorzelle eingebracht und exprimiert wird.

3. Verwendung nach Anspruch 1 oder 2, wobei das Gen, welches eine AMPA-Typ-Glutamatrezeptor-Untereinheit GluR2(R) codiert, eine cDNA einer AMPA-Typ-Glutamatrezeptor-Untereinheit GluR2(R) ist.

4. Verwendung nach einem der Ansprüche 1 bis 3, wobei das Gen, welches eine AMPA-Typ-Glutamatrezeptor-Untereinheit GluR2(R) codiert, durch einen Expressionsvektor in eine sich entwickelnde tierische Hirntumorzelle eingebracht wird und exprimiert wird.

5. Verwendung nach Anspruch 4, wobei der Expressionsvektor ein Vektor ist, welcher einen Adenovirus verwendet.

6. Verwendung nach einem der Ansprüche 1 bis 5, wobei die Hirntumorzelle ein Glioblastom ist.

7. Verfahren zum Messen der Proliferations-/Invasionsaktivität von Hirntumorzellen, wobei die Expression einer AMPA-Typ-Glutamatrezeptor-Untereinheit GluR2(R) in einer sich entwikkelnden tierischen Hirntumorzelle detektiert/gemessen wird.

8. Gen, welches eine AMPA-Typ-Glutamatrezeptor-Untereinheit GluR2(R) codiert, zur Verwendung beim Hemmen der *in vivo*-Proliferation und -Invasion von Hirntumorzellen durch Regulieren der Ca²⁺-Permeabilität durch eine AMPA-Typ-Glutamatrezeptor-Untereinheit GluR2(R), die in einer sich entwickelnden tierischen Hirntumorzelle exprimiert wird, wobei das Gen GluR2(R) ohne weiteres RNA-Editing codiert.

9. Gen für die Verwendung nach Anspruch 8, wobei das Gen, welches eine AMPA-Typ-Glutamatrezeptor-Untereinheit GluR2(R) codiert, in eine sich entwickelnde tierische Hirntumorzelle eingebracht und exprimiert wird.

10. Gen für die Verwendung nach Anspruch 8 oder 9, wobei das Gen, welches eine AMPA-Typ-Glutamatrezeptor-Untereinheit GluR2(R) codiert, eine cDNA einer AMPA-Typ-Glutamatrezeptor-Untereinheit GluR2(R) ist.

11. Gen für die Verwendung nach einem der Ansprüche 8 bis 10, wobei das Gen, welches eine AMPA-Typ-Glutamatrezeptor-Untereinheit GluR2(R) codiert, durch einen Expressionsvektor in eine sich entwickelnde tierische Hirntumorzelle eingebracht wird und exprimiert wird.

12. Gen für die Verwendung nach Anspruch 11, wobei der Expressionsvektor ein Vektor ist, welcher einen Adenovirus verwendet.

13. Gen für die Verwendung nach einem der Ansprüche 8 bis 12, wobei die Hirntumorzelle ein Glioblastom ist.

## Revendications

1. Utilisation d'un gène codant pour une sous-unité du récepteur de glutamate du type AMPA GluR2(R) pour la production d'un médicament destiné à l'inhibition de la prolifération et de l'invasion in vivo de cellules tumorales cérébrales, par régulation de la perméabilité au Ca²⁺ par une sous-unité du récepteur de glutamate de type AMPA GluR2(R) exprimée dans une cellule tumorale cérébrale d'animal en développement, dans laquelle le gène utilisé dans la production du médicament code pour GluR2(R) sans édition d'ARN supplémentaire.

2. Utilisation suivant la revendication 1, dans laquelle le gène codant pour une sous-unité du récepteur de glutamate de type AMPA GluR2(R) est introduit dans une cellule tumorale cérébrale d'animal en développement et est exprimé.

3. Utilisation suivant la revendication 1 ou 2, dans lequel le gène codant pour une sous-unité du récepteur de glutamate de type AMPA GluR2(R) est un ADNc d'une sous-unité du récepteur de glutamate de type AMPA GluR2(R).

4. Utilisation suivant l'une quelconque des revendications 1 à 3, dans laquelle le gène codant pour une sous-unité du récepteur de glutamate de type AMPA GluR2(R) est introduit dans une cellule tumorale cérébrale d'animal en développement par un vecteur d'expression et est exprimé.

5. Utilisation suivant la revendication 4, dans laquelle le vecteur d'expression est un vecteur utilisant un adénovirus.

6. Utilisation suivant l'une quelconque des revendications 1 à 5, dans lequel la cellule tumorale cérébrale est un glioblastome.

7. Méthode pour mesurer l'activité de prolifération/invasion de cellules tumorales cérébrales, dans laquelle l'expression d'une sous-unité du récepteur de glutamate de type AMPA GluR2(R) dans une cellule tumorale cérébrale d'animal en développement est détectée/mesurée.

8. Gène codant pour une sous-unité du récepteur de glutamate de type AMPA GluR2(R), destiné à être utilisé dans l'inhibition de la prolifération et de l'invasion in vivo de cellules tumorales cérébrales par régulation de la perméabilité au Ca²⁺ par une sous-unité du récepteur de glutamate de type AMPA GluR2(R) exprimée dans une cellule tumorale cérébrale d'animal en développement, où le gène code pour GluR2(R) sans édition d'ARN supplémentaire.

9. Gène destiné à être utilisé suivant la revendication 8, où le gène codant pour une sous-unité du récepteur de glutamate de type AMPA GluR2(R) est introduit dans une cellule tumorale cérébrale d'animal en développement et est exprimé.

10. Gène destiné à être utilisé suivant la revendication 8 ou 9, où le gène codant pour une sous-unité du récepteur de glutamate de type AMPA GluR2(R) est un ADNc d'une sous-unité du récepteur de glutamate de type AMPA GluR2(R).

11. Gène destiné à être utilisé suivant l'une quelconque des revendications 8 à 10, où le gène codant pour une sous-unité du récepteur de glutamate de type AMPA GluR2(R) est introduit dans une cellule tumorale cérébrale d'animal en développement par un vecteur d'expression et est exprimé.

12. Gène destiné à être utilisé suivant la revendication 11, le vecteur d'expression étant un vecteur utilisant un adénovirus.

13. Gène destiné à être utilisé suivant l'une quelconque des revendications 8 à 12, la cellule tumorale cérébrale étant un glioblastome.


### SEQUENCE LISTING

<110> Japan Science and Technology Agency
<120> Inhibition of Proliferation and Infiltration of Brain Tumor Cells Caused by Expression of Ampa-Type Glutamate Receptor Subunit
<130> P022437EP CTH
<140> EP03784615.1
   <141> 2003-08-08
<150> JP P2002-232086
   <151> 2002-08-08
<150> PCT/JP03/10143
   <151> 2003-08-08
<160> 6
<170> PatentIn version 3.0
<210> 1
   <211> 26
   <212> DNA
   <213> Artificial
<220>
   <223> PCR primer
<400> 1
   cctttggcct atgagatctg gatgtg 26
<210> 2
   <211> 22
   <212> DNA
   <213> Artificial
<220>
   <223> PCR primer
<400> 2
   tcgtaccacc atttgttttt ca 22
<210> 3
   <211> 22
   <212> DNA
   <213> Artificial
<220>
   <223> PCR primer
<400> 3
   aagagggacg agaccagaca ac 22
<210> 4
   <211> 24
   <212> DNA
   <213> Artificial
<220>
   <223> PCR primer
<400> 4
   gaagatggaa gagaaacaca aagt 24
<210> 5
   <211> 23
   <212> DNA
   <213> Artificial
<220>
   <223> PCR primer
<400> 5
   ggaagacaac aatgaagaac ctc 23
<210> 6
   <211> 20
   <212> DNA
   <213> Artificial
<220>
   <223> PCR primer
<400> 6
   gaaggaccca gcgaccagcc 20